(19) Europäisches Patentamt
European Patent Office
Office européen des brevets

(11) Publication number: **0 244 778 B1**

(12) # EUROPEAN PATENT SPECIFICATION

(45) Date of publication of patent specification: **16.10.91** (51) Int. Cl.$^5$: **C07C 265/12**

(21) Application number: **87106322.8**

(22) Date of filing: **30.04.87**

The file contains technical information submitted after the application was filed and not included in this specification

(54) **Mono-tertiary-alkylated toluenediiso-cyanates and derivatives.**

(30) Priority: **06.05.86 US 860283**

(43) Date of publication of application:
**11.11.87 Bulletin 87/46**

(45) Publication of the grant of the patent:
**16.10.91 Bulletin 91/42**

(84) Designated Contracting States:
**BE CH DE FR LI SE**

(56) References cited:
**EP-A- 0 125 591**
**DD-A- 146 043**
**US-A- 2 963 504**

(73) Proprietor: **AIR PRODUCTS AND CHEMICALS, INC.**
**Route no. 222**
**Trexlertown Pennsylvania 18087(US)**

(72) Inventor: **Dixon, Dale D.**
**R.D No 3, Box 337-35**
**Kutztown, PA 19530(US)**
Inventor: **Burgoyne, William F., Jr.**
**1950 Potomac St.**
**Allentown, PA 18103(US)**
Inventor: **Casey, Jeremiah P.**
**2665 Arbor Circle**
**Emmaus. PA 18049(US)**
Inventor: **Milligan, Barton**
**R.D. No. 1**
**Coplay, PA 18037(US)**

(74) Representative: **Schwabe - Sandmair - Marx**
**Stuntzstrasse 16**
**W-8000 München 80(DE)**

**EP 0 244 778 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention pertains to mono-tertiary alkyl derivatives of toluenediisocyanates

## BACKGROUND OF THE INVENTION

Alkylated aromatic diisocyanates and blocked isocyanates have been known for a substantial period of time and find use in the preparation of polyurethane and polyurea elastomers. Toluenediisocyanate and biphenyl diisocyanates are widely used in the manufacture of such polyurethane and polyurea elastomers and are formulated into such elastomers by techniques such as RIM manufacturing and cast systems. The RIM method of formulating polyurethane and polyurea elastomers is becoming of extreme importance by virtue of ability to formulate the elastomers in a "one shot" method, wherein the isocyanate, organic compound having active hydrogen atoms, e.g., a long chain polyol or an amine terminated polyol along with a chain extender suitably an aromatic diamine chain extender. Some of the isocyanate systems are relatively fast acting and it is desirable to obtain various isocyanates having different reactivities so that greater flexibility can be achieved in the molding process.

Aromatic isocyanates are commonly prepared by synthesizing an aromatic diamine and then reacting the aromatic diamine with phosgene to form the diisocyanate. Accordingly, toluenediisocyanate is formed by reacting toluenediamine with phosgene and methylene di(phenylamine) is reacted with phosgene to form methylene di(phenylisocyanate). Representative patents illustrating various alkyl aromatic diisocyanates and derivatives thereof are as follows:

U.S. 2,963,504 discloses a 2,6-diisocyanato-4-tert-butyltoluene composition which was formed from the 2,6-diamino-4-tert-butyltoluene derivative. No synthesis technique was shown. However, from the disclosed isomer structure, the product was produced by alkylating toluene, and then dinitrating the alkylated product followed by reducing the nitro group to amine.

U.S. 2,757,184 discloses a process for preparing substituted di(isocyanatophenyl) ureas. The isocyanates disclosed in the patent contain two phenyl groups and substituent groups, the substituted groups are ortho to the isocyanate group and are represented as being an alkyl group, alkoxy radical or a chlorine atom. Representative of alkyl groups include methyl, ethyl, propyl, butyl, and hexyl, while the alkoxy radicals are methoxy, ethoxy, butoxy and the like. Such diisocyanates apparently have a higher melting point than the mono nuclear counterparts and a low vapor pressure, making them much less hazardous to handle than toluenediisocyanate for example and they possess lower reactivity.

U.S. 3,071,557 discloses the preparation of segmented polymers which contain a repeating unit derived from an alkyl substituted aromatic diisocyanate, such as 2,4,6-trimethyl-1,3 phenylenediisocyanate and 2,4,6-triisopropyl-1,3-phenylenediisocyanate.

U.S. 3,351,650 discloses the manufacture of mixed isomers of lower alkyl cyclohexylene polyisocyanates and polyurethane systems prepared from these polyisocyanates. The lower alkyl cyclohexylene polyisocyanates prepared from the corresponding lower alkyl cyclohexylene diamine. Examples of alkyl substituents include the ethyl and propyl radical.

U.S. 3,203,931 discloses the use of 4-substituted toluene diisocyanates for the manufacture of urethanes. Representative substituted toluenediisocyanates with the substituent being in the 4 position, include alkyl, ether, ketone, nitrile, and amine. Examples of substituted aromatic diisocyanates include the para-bromotoluene; 4-butoxytoluene; and para-butyltoluene diisocyanate. These diisocyanates are reacted with a polyester or polyether polyol to form a prepolymer which is then further reacted to form cellular polyurethane systems. The para-tert-butyl-2,6-toluenediisocyanate was alleged to have good inhibiting action to surface darkening and light resistance in polyurethane compositions used for coatings, foams and elastomers.

## SUMMARY OF THE INVENTION

This invention pertains to mono-tert-alkyltoluenediisocyanates and derivatives having a specific isomeric structure. In contrast to the prior art toluenediisocyanate compositions, the tertiary alkyl group is ortho to an isocyanate group and the alkyl group contains a tertiary carbon atom. These compositions are best represented by the formulas:

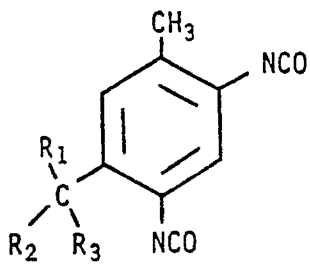

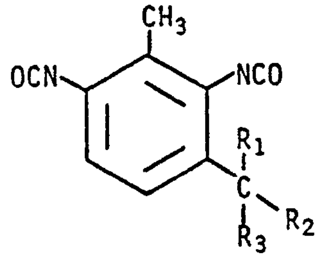

wherein $R_1$, $R_2$ and $R_3$ are $C_{1-3}$ alkyl groups or $R_2$ and $R_3$ are combined to form a $C_{5-6}$ membered ring.

There are several advantages associated with the specific isomeric compounds of this invention. One advantage is that the mono-tert-alkylated-2,6-toluenediisocyanate has a reactivity which is slower than toluenediisocyanate and many other isocyanates, thus making that isomer more acceptable for manufacturing a variety of large parts. Another is that the reactivity of one isocyanate group is rendered substantially less reactive by the tert-carbcn containing alkyl group than the other isocyanate on the ring, thus providing a mechanism for a selective reaction without the use of a contaminating non reactive component as encountered with blocked isocyanate. A third advantage is that elastomers requiring extended potlife for molding can be formulated with these isocyanates.

## DETAILED DESCRIPTION OF THE INVENTION

The compounds of this invention as indicated above, are represented by the formulas:

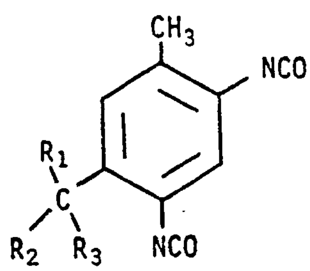

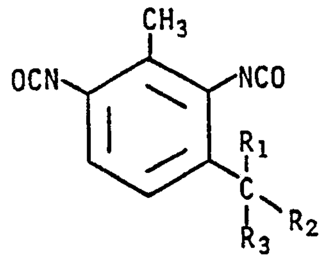

wherein $R_1$, $R_2$ and $R_3$ are $C_{1-3}$ alkyl groups or $R_2$ and $R_3$ are combined to form a $C_{5-6}$ membered ring.

The primary differences between the compositions described herein and those in the prior art, are: the compositions are mono alkyl substituted toluenediisocyanate derivatives containing an alkyl group ortho to one isocyanate group and the alkyl group contains a tertiary carbon atom, e.g. a t-butyl radical. As is shown, the toluenediisocyanates are either the 2,4-toluenediisocyanate or 2,6-toluenediisocyanate. The methyl radical is in the 1 position.

The compounds of this invention can be synthesized by alkylating toluenediamine compounds as opposed to the technique of alkylating an aromatic hydrocarbon, e.g., toluene followed by amination through a nitration route. It is because the alkylation is effected on toluenediamine that one obtains a mono-alkyl group having a tertiary carbon atom ortho to an isocyanate group in the aromatic composition. When alkylation is first effected on an aromatic hydrocarbon as opposed to a toluenediamine, the alkyl group is directed to the position para to the methyl group (meta to the amine and hence meta to the isocyanate produced from the amine). Only in the case of European 69,286 where there is at least two alkyl (methyl) groups can one obtain an composition having an alkyl group ortho to an amino group. On the other hand, the amine groups in toluenediamine are ortho directing and therefore the mono-alkyl group becomes ortho to an amino group and thus ortho to the isocyanate.

The art has utilized heterogeneous catalyst systems to effect alkylation of aromatic amines and examples of these catalytic systems include silica-alumina, montmorillonite, kaolin crystalline molecular sieves and zeolites. Of these, crystalline zeolites and crystalline molecular sieves appear to offer advantages in that they have desired acidity and they can accommodate selective production of the mono-tert-alkyltoluenediamine for conversion to the diisocyanate product. Crystalline zeolites are well known and basically are alumino-silicates where the mole ratio of silica to alumina is very high, e.g., greater than 3:1.

Such zeolites can be substituted with various cations to alter their acidity and thus their activity with respect to achieving alkylation of toluenediamine. These cations can also alter the effective pore size of the zeolite and thus alter their catalytic activity. Two common types of zeolites are the X zeolite and the Y zeolite with the Y zeolite typically having a higher silica content. Typically a Y zeolite will have a silica to alumina molar ratio of about 3:1. Although the X and Y zeolites are common examples of zeolites suited for practicing the invention, others include faujasite, offretite, chabazite, L, omega, and the ZSM family. The Y zeolite or faujasite because of its silica to alumina ratio and pore configuration is well suited for the production of many mono-tert-alkyltoluenediamine products. This is particularly true with respect to the reaction of isobutylene with toluenediamine to produce mono-tert-butyltoluenediamine.

As noted, the acidity of zeolites can be controlled by utilizing various cations in place of the sodium atom in a synthetic or naturally occurring zeolite. The preferred cations suited for the catalyst system include the rare earth metals, such as, lanthanum, cerium, praseodymium, as well as various transition metals and hydrogen. Basic metal exchanged zeolites, which render the resulting zeolite weakly acidic, are relatively inactive. Alkali metals, in particular, decrease the reactivity of the exchanged zeolite. For this reason the highly or strongly acidic zeolites, i.e., hydrogen, rare earth metal exchange or those with a high silica to alumina ratio are preferred.

The pore size of zeolites has an influence on the reactivity and type of products produced. They may be altered toward either larger or smaller sizes by variety of known means. For example, it is known that pore sizes may be reduced by chemical modification, e.g. addition of oxides of boron and phosphorus, or coke formation. It is therefore possible to render many zeolitic systems capable of synthesizing products having large molecular sizes by increasing the effective pore size of the zeolite from that in its unaltered state. The primary method of enlarging pore size is through removing alumina from the catalyst structure. Some of the main techniques for removing alumina are hydrothermal treatment (steaming) and acid washing. Thus, a zeolite which may have insufficient pore size to accommodate entry of the molecules for reaction may be expanded to have an effective pore size that will accommodate entry of such molecules. Alternatively, if the pore size is too large and substantial amounts of the di-tert-alkylated-toluenediamine is produced, one can reduce the effective pore size of the zeolite by the introduction of large metal cations. However, the preferred technique is to select a zeolite having a smaller effective pore size to limit synthesis of the alkylated toluenediamines having larger molecular sizes.

The mono-tert-alkyltoluenediamines are prepared by reacting an olefin with toluenediamine in the presence of an acidic acid catalyst as described. Examples of olefins for producing a toluenediamine derivative having a tertiary-carbon atom include isobutylene, isoamylene, 2-ethyl-1-pentene, 2-ethyl-2-pentene, 2-methyl-1-pentene, 2-methyl-2-pentene, isooctene, and 1-methylcyclohexene. The relationship of the branched chain and olefin must be such that a tertiary carbon is available for alkylation. In this way $R_1$, $R_2$ and $R_3$ can have from 1 to 3 carbon atoms or $R_2$ and $R_3$ can be combined to form a 5 or 6 membered ring.

Ring alkylation of the 2,4- and 2,6-toluenediamines is effected by reacting the toluenediamine with the olefin or olefin precursor such as a tertiary-alkanol. In the case where the olefin is generated from an olefin precursor such as an alcohol, it is believed that such olefin is formed, in situ, by a dehydration of the alcohol. Typically, water, which is a by product from such reaction, tends to deactivate the catalytic system and temperature modifications may be required. Typically, where a 1,1-disubstituted olefin is used for the alkylation reaction, temperatures will range from about 100 to 250 °C; preferably, 160 to 200 °C and the pressures will range from about 15 to 2000 psig and generally in the range of 100 to 1000 psig. It is common practice to alter the temperature and pressure within the above ranges specified to optimize the selectivity and conversion to the desired product. Mole ratios of olefin to toluenediamine used in the reaction will range from about 1 to 10:1 and the reaction time will generally be from about 2 to 48 hours. Where fixed bed catalytic reactors are used, the feed to the reactor, expressed as an LHSV, will range from about 0.05-6 $hours^{-1}$.

The alkylated toluenediisocyanates are prepared from the corresponding alkyl substituted toluenediamine by well-known techniques. Typically, this is accomplished by reacting the alkylated aromatic diamine with phosgene. This is a conventional procedure wherein the aromatic amine is dispersed in a solvent and phosgene gas introduced to the solvent mixture containing aromatic diamine and heated. Phosgenation of the amine to form the isocyanate is carried out at reflux conditions with a reaction time from 2-10 hours. After completion of the reaction, the solution is cooled and an inert gas such as nitrogen passed through the system to remove residual phosgene. The product diisocyanate then is recovered by conventional techniques, usually by distillation.

The alkylated toluenediisocyanates may be used by themselves, as a blend, e.g. a mixture of the 2,4- and 2,6-isomers, respectively, and the isomers may be used in combination with other aromatic

diisocyanates and alkylated diisocyanates. A mixture of the 2,4-isomer and 2,6-isomer in a weight ratio of from about 65-80% of the 2,4, 20-35% of the 2,6-isomer and optionally 0-5% of the vicinal isomers is attractive for a number of reasons. One reason is that a commercial feedstock of toluenediamine, after separation of the vicinal toluenediamine, typically contains about 80% of the 2,4-isomer and 20% of the 2,6-isomer. Another commercial route to toluenediamine produces an isomer ratio of about 65% of the 2,4- and 35% of the 2,6-isomer. The mixture can be alkylated in the same manner as the individual isomers.

A second advantage of the mixture of diisocyanates is in the reactivity difference associated therewith as compared to the individual isomers.

The following examples are provided to illustrate embodiments of the invention for synthesizing alkylated aromatic amines the conversion of the alkylated aromatic diamines to diisocyanates and to illustrate performance of these alkylated diisocyanates in the manufacture of polyurethane resins.

Example 1

Alkylation of a 80:20 Mixture of the 2,4- and 2,6-isomers of toluenediamine with Isobutylene over H-Y zeolite

A 15.0 g. portion of H-Y zeolite (powder) 120.0 g. (0.98 mol) of 2,4-toluenediamine, and 30.0 g. (0.25 mol) of 2,6-toluenediamine were charged to a 1000 cc Hastalloy C pressure vessel equipped with a mechanical stirrer. The vessel was sealed and purged with nitrogen, leaving a 217 psig nitrogen blanket. The contents were heated to 180 °C with stirring. Isobutylene (280g., 4.98 mol) was then added over 15 minutes resulting in an initial reaction pressure of 1271 psig. The reaction mixture was maintained at 180 °C for 18 hours with constant stirring and then cooled to 150 °C. Stirring was discontinued at this time and the residual pressure vented. Upon removal of the catalyst by hot filtration, a product mixture of the following composition was obtained:

| | Mole% |
|---|---|
| 2,4-toluenediamine | 19.09 |
| 2,6-toluenediamine | 6.30 |
| 2-(tert-butylamino)-4-aminotoluene | 2.03 |
| 2-amino-4-(tert-butylamino)toluene | 8.11 |
| 5-tert-butyl-2,4-toluenediamine | 48.79 |
| 3-tert-butyl-2,6-toluenediamine | 12.73 |
| 2-(tert-butylamino)-5 tert-butyl-4-aminotoluene | 1.60 |
| 2-amino-5-tert-butyl-4-(tert-butylamino)toluene | 0.55 |
| 2-(tert-butylamino)-5-tert-butyl-6-aminotoluene | trace |
| 3,5-di-tert-butyl-2,6-toluenediamine | 0.81 |

Example 2

Preparation of 5-tert-butyl-2,4-toluenediamine

A 15.0 g. portion of powdered H-Y zeolite and 150.0g. (1.23 mol) of 2,4-toluenediamine were charged to a 1000 cc. Hastalloy C pressure vessel equipped with a mechanical stirrer. The vessel was sealed and purged with nitrogen leaving a residual 225 psig nitrogen blanket. The vessel contents were heated to 180 °C with stirring at 500 rpm. Isobutylene (279.0 g., 4.98 mol) was then added over 2 hours, resulting in 1225 psig vessel pressure. This provided a mole ratio of 4.05 moles isobutylene to 1 mole toluenediamine. The reaction mixture was maintained at 180 °C for 16 hours with constant stirring. The contents were cooled to 150 °C and then stirring was discontinued and the residual pressure vented. Removal of the catalyst by hot filtration afforded the following product mixture:

| | Mole% |
|---|---|
| 2,4-toluenediamine | 15.59 |
| 2-(tert-butylamino)-4-aminotoluene | 1.66 |
| 2-amino-4-(tert-butylamino)toluene | 8.02 |
| 5-tert-butyl-2,4-toluenediamine | 71.60 |
| 2,4-di(tert-butylamino)toluene | 0.20 |
| 2-(tert-butylamino)-5-tert-butyl-4-aminotoluene | 1.38 |
| 2-amino-5-tert-butyl-4-(tert-butylamino)toluene | 0.55 |

Example 3
Preparation of 3-tert-butyl-2,6-toluenediamine

A 15.0 g. portion of powdered H-Y zeolite catalyst and 140.0 g. (1.15 mol) of 2,6-toluenediamine were charged to a 1000 cc Hastalloy C pressure vessel equipped with a mechanical stirrer as was done in Example 2. The vessel was sealed and purged with nitrogen leaving a residual 200 psig nitrogen blanket at room temperature. The contents were heated to 180 °C with stirring. Isobutylene (267 g., 4.76 mol) was then added to the reaction mixture over 20 minutes, resulting in an initial reaction pressure of 1100 psig. This provided a molar ratio of 4.1:1 isobutylene to toluenediamine. The reaction mixture was maintained at 180 °C for 39 hours with constant stirring. The reaction product was isolated by the procedure used in Example 1 and consisted of the following composition:

| | Mole % |
|---|---|
| 2,6-toluenediamine | 30.48 |
| 2-(tert-butylamino)-6-aminotoluene | 9.79 |
| 3-tert-butyl-2,6-toluenediamine | 56.13 |
| 2-(tert-butylamino)-5-tert-butyl-6-aminotoluene | 1.19 |
| 3,5-di-tert-butyl-2,6-toluenediamine | 1.28 |

Example 7

A series of alkylation products was prepared in accordance with the general procedures outlined in Examples 1-6 using various catalyst systems under a variety of conditions. These reactions A-L are set forth in Table 1. For convenience the term alkyl has been used in describing specific alkylated products designated in the product distribution portion of the table.

Table I

Alkylation of 2,6-toluenediamine with Di- and Trisubstituted Olefins

| Entry | A | B | C | D |
|---|---|---|---|---|
| olefin | 2-methyl-2-butene | 2-methyl-2-butene | 2-methyl-2-butene | 2-methyl-2-butene |
| catalyst | 12 M HCl | 18 M $H_2SO_4$ | 13% $Al_2O_3$/87% $SiO_2$ | H-Y Zeolite |
| moles olefin/moles amines | 2 | 2 | 2.7 | 2 |
| grams amine/grams catalyst | 10 | 10 | 10 | 10 |
| temperature/Initial Total Pressure | 200°C/750 psig | 200°C/650 psig | 200°C/1690 psig | 180°C/440 psig |
| reaction Time | 20 hr | 23 hr | 22 hr | 19 hr |
| Product Distribution (wt %)* | | | | |
| 2,6-toluenediamine | 83.18 | 81.06 | 89.36 | 90.61 |
| N-*tert*-alkyl-2,6-toluene-diamine | 1.54 | 2.45 | 1.55 | 1.93 |
| 3-*tert*-alkyl-2,6-toluenediamine | 10.11 | 15.17 | 8.35 | 5.97 |
| di-*tert*-alkyl-2,6-toluenediamine | 5.17 | 1.32 | 0.74 | 1.49 |

*Analysis performed on an olefin-free sample

Table I (cont'd.)

Alkylation of 2,6-toluenediamine with di- and trisubstituted Olefins

| Entry | E | F | G |
|---|---|---|---|
| olefin | 2-methyl-1-butene | 1-methylcyclopentene | 1-methylcyclohexene |
| catalyst | 13% $Al_2O_3/SiO_2$ | 13% $Al_2O_3/SiO_2$ | H-Y Zeolite |
| moles olefin/moles amine | 1.74 | 1.48 | 2.0 |
| grams amine/grams catalyst | 12.50 | 10 | 8.13 |
| temperature/Initial Total Pressure | 200°C/602 psig | 200°C/211 psig | 180°C/250 psig |
| reaction Time | 12.5 | 18 hr | 19 hr |
| <u>Product Distribution (wt %)*</u> | | | |
| 2,6-toluenediamine | 80.78 | 75.14 | 99.99 |
| <u>N</u>-<u>tert</u>-alkyl-2,6-toluenediamine | 2.30 | 1.07 | -- |
| 3-<u>tert</u>-alkyl-2,6-toluenediamine | 16.07 | 23.58 | trace |
| di-<u>tert</u>-alkyl-2,6- | 0.85 | 0.21 | -- |

*Analysis performed on an olefin-free sample

Table 1 (Cont'd.)

Alkylation of 2,4-toluenediamine with di- and trisubstituted Olefins

| Entry | H | I | J |
|---|---|---|---|
| olefin | 2-methyl-2-butene | 2-methyl-1-butene | 1-methyl-cyclopentene |
| catalyst | H-Y zeolite | 13% $Al_2O_3$/87% $SiO_2$ | 13% $Al_2O_3$/87% $SiO_2$ |
| moles olefin/ moles amine | 2 | 1.75 | 1.48 |
| grams amine/ grams catalyst | 10 | 12.5 | 10 |
| temperature/Initial Total Pressure | 180°C/450 psig | 200°C/495 psig | 200°C/187 psig |
| reaction time | 22 hr | 19 hr | 22 hr |
| Product Distribution (wt %)* | | | |
| 2,4-toluenediamine | 88.45 | 77.89 | 77.86 |
| 2-*N*-*tert*-alkyl-2,4-toluenediamine | 1.12 | 1.96 | 0.69 |
| 4-*N*-*tert*-alkyl-2,4-toluenediamine | 3.92 | 8.34 | 2.66 |
| 5-tert-alkyl-2,4-toluenediamine | 6.51 | 11.81 | 18.68 |
| di-*tert*-alkyl-2,4-toluenediamine | -- | -- | -- |

*Analysis performed on an olefin-free sample

Table 1 (cont'd.)

Alkylation of 2,4-toluenediamine with di- and trisubstituted Olefins

| Entry | K | L |
|---|---|---|
| olefin | 2-methyl-1-pentene | 2,3-dimethyl-1-butene |
| catalyst | 13% $Al_2O_3$/87% $SiO_2$ | 13% $Al_2O_3$/87% $SiO_2$ |
| moles olefin/ moles amine | 2 | 2 |
| grams amine/ grams catalyst | 15 | 10 |
| temperature/Initial Total Pressure | 198°C/295 psig | 200°C/315 psig |
| reaction Time | 24 hr | 96 hr |
| Production Distribution (wt %)* | | |
| 2,4-toluenediamine | 74.45 | 88.76 |
| 2-N-*tert*-alkyl-2,4-toluenediamine | trace | --- |
| 4-N-*tert*-alkyl-2,4-toluenediamine | 4.69 | 2.51 |
| 5-*tert*-alkyl-2,4-toluenediamine | 18.69 | 4.92 |
| di-*tert*-alkyl-2,4-toluenediamine | 2.17 | --- |

*Analysis performed on an olefin-free sample

Example 8

Preparation of 5-tert-butyl-2,4-toluenediisocyanate

Diphosgene (220 g, 1.1 mole) was charged to a 2 liter single neck round bottom flask, equipped with a thermowell, addition funnel, a condenser with Claisen adapter, magnetic stir bar and a nitrogen purge stream. 5-tert-Butyl-2,4-toluenediamine (96.1 g, 0.54 mole) in 909.5 g of dioxane was added dropwise over a period of one hour. During the addition the temperature rose from 18° to 37° C and a yellow precipitate formed. On completion of the addition the slurry was heated to 70° C for 3.5 hours and then allowed to cool to room temperature. The condenser was replaced with a distillation head and dioxane and excess diphosgene were removed via distillation with the oil bath not exceeding 140° C. The residue was flash-distilled at 125-140° C under aspirator vacuum affording a 95% yield (117.4 g; 0.51 mole) of 5-tert-butyl-2,4-toluenediisocyanate.

Example 9

Preparation of 3-tert-butyl-2,6-toluenediisocyanate

Diphosgene (12.2 g, 0.06 mole) was charged to a 100 ml 3-neck round bottom flask equipped with a thermometer, addition funnel, condenser, magnetic stir bar and nitrogen purge stream. 3-tert-Butyl-2,6-toluenediamine (5.5 g, 0.031 mole) in 50 g of dioxane was added dropwise over a period of 20 minutes. During the addition the temperature rose from 20° C to 38° C and a violet precipitate formed. On completion of the addition the slurry was heated to 60° C for 2.5 hours and then allowed to cool to room temperature.

The condenser was replaced with a distillation head and dioxane and excess diphosgene were removed via distillation with the oil bath not exceeding 140° C. The residue was flash-distilled at 125-140° C under aspirator vacuum affording a 92.3% yield (6.5 g; 0.028 mole) 3-tert-butyl-2,6-toluenediisocyanate.

The products prepared by examples 8 and 9 were identified by $^1$H-NMR, $^{13}$C-NMR, I.R., mass spectroscopy and elemental analysis.

Example 10

This example demonstrates that 3-tert-butyl-2,6-toluenediisocyanate (3tBTDI) may be selectively reacted with an alcohol at one isocyanate position while leaving the other available for later reaction with amines. The control 2,6-toluenediisocyanate, on the other hand, shows significantly less selectivity and tends to react at both isocyanate groups when in the presence of excess alcohol.

To a 15 ml vial containing a magnetic stir bar was added 0.1 m.mole of diisocyanate (vial 1 = 0.15 g TDI; vial 2 = 0.2 g 3tBTDI), 0.8 g tetrahydrofuran and 0.21 g of isopropanol. A nitrogen blanket was maintained by use of a septum seal. The mixture was monitored by gas chromatography. At a reaction time of 96 hours the 3-tert-butyl-2,6-toluenediisocyanate showed a 93% selectivity for reaction at one isocyanate group whereas the control 2,6-toluenediisocyanate showed only a 70% selectivity for reaction at one isocyanate group. The latent reactivity of the 3-tert-butyltoluenediisocyanate as compared to 2,6-toluenediisocyanate permits one to formulate a multitude of elastomer systems requiring extended potlife for molding.

Example 11

This example demonstrates the utility of 3-tert-butyl-2,6-toluenediisocyanate in forming polymers possessing pendant isocyanate groups for latent crosslinking useful in applications such as adhesives, coatings and elastomers. The control, 2,6-toluenediisocyanate, has two reactive isocyanate groups and undergoes crosslinking in an uncontrolled manner.

(a) Reaction of 2,6-toluenediisocyanate with poly-co-vinylacetate-co-vinylalcohol.

2,6-Toluenediisocyanate (0.34 g) was added to a beaker containing 3.4 g of poly-co-vinylacetate-co-vinylalcohol (45% hydrolyzed) in 6.5 g dimethylformamide. Addition of six drops of triethylamine catalyst resulted in an immediate increase in viscosity. After 10 min. the solution was converted to a gum, indicating that crosslinking had occurred.

(b) Reaction of 3-tert-butyl-2,6-toluenediisocyanate with poly-co-vinylacetate-co-vinylalcohol.

3-tert-butyl-2,6-toluenediisocyanate (0.34 g) was added to a beaker containing 3.4 g of poly-co-vinylacetate-co-vinylalcohol (45% hydrolyzed) in 6.5 g dimethylformamide. Addition of six drops of triethylamine catalyst had no visible effect on the solution viscosity. The solution viscosity remained unchanged for 50 minutes and then diethylenetriamine (0.1 g) was added to the solution which then quickly turned to a viscous gum. The observations of this experiment indicate that the initial reaction formed a polymer having a pendant isocyanate which did not readily crosslink with hydroxyl groups but was crosslinkable upon addition of diamines.

Experiment 12

Samples prepared in Example 11 were drawn down on glass panels which were then placed in a vacuum oven at 80° C for 3.5 hours to remove residual dimethylformamide. The resulting films and substrate plates were then immersed in boiling water for 45 minutes. The only film still adhering to the glass surface was the film formed by crosslinking the pendant isocyanate group with diethylenetriamine.

| Film Sample | Conditions After 45 min. in boiling $H_2O$ |
|---|---|
| poly-co-vinylacetate-co-vinylalcohol (control) | dissolved |
| 2,6-toluenediisocyanate/ poly-co-vinylacetate-co-vinylalcohol (control) | removed from plate |
| 3-tert-butyl-2,6-toluenediisocyanate/ poly-co-vinylacetate-co-vinylalcohol (uncrosslinked) | dissolved |
| 3-tert-butyl-2,6-toluenediisocyanate/ poly-co-vinylacetate-co-vinylalcohol (crosslinked by addition of diethylenetriamine) | intact on panel |

## Claims

1. An alkyl substituted toluenediisocyanate composition wherein said alkyl substituent has a tertiary carbon atom said composition represented by the formulas:

1

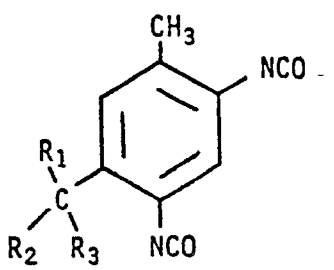

2

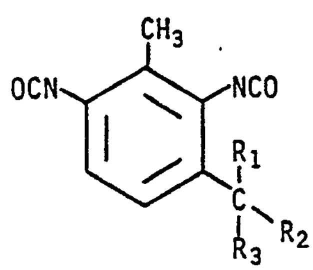

wherein $R_1$, $R_2$ and $R_3$ are $C_{1-3}$ alkyl groups or $R_2$ and $R_3$ are combined to form $C_{5-6}$ membered ring.

2. The composition of Claim 1 wherein $R_1$, $R_2$ and $R_3$ are $C_{1-3}$ alkyl groups.

3. The composition of Claim 2 wherein said composition is 2,6-diisocyanato-3-tert-butyltoluene or 2,4-diisocyanato-5-tert-butyltoluene.

4. The composition of Claim 2 containing approximately 65-80% by weight of the 2,4-diisocyanato-5-tert-butyltoluene and 20-35% by weight of the 2,6-diisocyanato-3-tert-butyltoluene.

5. The composition of Claim 4 wherein the composition contains approximately 80% by weight of 2,4-diisocyanato-5-tert-butyltoluene and 20% by weight of 2,6-diisocyanato-3-tert-butyltoluene.

6. The composition of Claim 2 wherein $R_1$ is ethyl and $R_2$ is methyl or ethyl and $R_3$ is a methyl group.

7. The composition of Claim 6 wherein $R_1$ is ethyl $R_2$ is ethyl and $R_3$ is methyl.

8. The composition of Claim 2 wherein $R_1$ is methyl and $R_2$ and $R_3$ are methyl or ethyl groups.

9. The composition of Claim 2 wherein $R_1$ is ethyl, $R_2$ is methyl or ethyl and $R_3$ is methyl.

10. The composition of Claim 1 wherein $R_1$ is $C_{1-3}$ and $R_2$ and $R_3$ are combined into a ring having from 5-6 carbon atoms and the composition contains from about 65-80% by weight of formula 1 and about 20-35% by weight of formula 2.

11. The composition of Claim 10 wherein $R_2$ and $R_3$ are combined to form a 5 carbon membered ring.

12. The composition of Claim 11 wherein $R_1$ is $C_1$.

13. The composition of Claim 10 wherein $R_2$ and $R_3$ are combined and represent a six carbon membered ring.

14. The composition of Claim 9 wherein $R_1$ is $C_1$.

## Revendications

1. Une composition de diisocyanate de toluène alkylsubstitué caractérisée en ce que le substituant alkyle possède un atome de carbone tertiaire, ladite composition étant représentée par les formules:

1

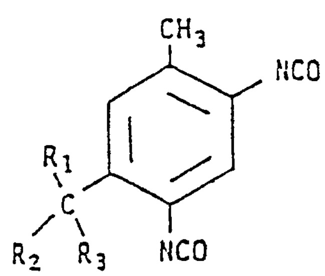

2

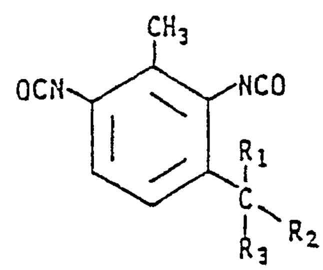

dans lesquelles $R_1$, $R_2$ et $R_3$ sont des radicaux alkyle $C_{1-3}$ ou $R_2$ et $R_3$ sont combinés pour former un cycle $C_{5-6}$.

2. La composition de la revendication 1 caractérisée en ce que $R_1$, $R_2$ et $R_3$ sont des radicaux alkyle $C_{1-3}$.

3. La composition de la revendication 1 caractérisée en ce que ladite composition est du 2,6-diisocyanato-3-tert-butyltoluène ou du 2,4-diisocyanato-5-tert-butyltoluène.

4. La composition de la revendication 2 caractérisée en ce qu'elle contient approximativement 65 à 80 % en poids de 2,4-diisocyanato-5-tert-butyltoluène et 25 à 35 % en poids de 2,6-diisocyanato-3-tert-butyltoluène.

5. La composition de la revendication 4 caractérisée en ce que la composition contient approximativement 80 % en poids de 2,4-diisocyanato-5-tert-butyltoluène et 20 % en poids de 2,6-diisocyanato-3-tert-butyltoluène.

6. La composition de la revendication 2 caractérisée en ce que $R_1$ est un radical éthyle, $R_2$ un radical méthyle ou éthyle et $R_3$ un radical méthyle.

7. La composition de la revendication 6 caractérisée en ce que $R_1$ est un radical éthyle, $R_2$ un radical éthyle et $R_3$ un radical méthyle.

8. La composition de la revendication 2 caractérisée en ce que $R_1$ est un radical méthyle et $R_2$ et $R_3$ des radicaux méthyle ou éthyle.

9. La composition de la revendication 2 caractérisée en ce que $R_1$ est un radical éthyle, $R_2$ un radical méthyle ou éthyle et $R_3$ un radical méthyle.

10. La composition de la revendication 1 caractérisée en ce que $R_1$ est $C_{1-3}$ et $R_2$ et $R_3$ sont combinés pour former un cycle comportant 5 à 6 atomes de carbone et en ce que la composition contient environ 65 à 80 % en poids de la formule 1 et environ 20 à 35 % en poids de la formule 2.

11. La composition de la revendication 10 caractérisée en ce que $R_2$ et $R_3$ sont combinés pour former un cycle à 5 atomes de carbone.

12. La composition selon la revendication 11 caractérisée en ce que $R_1$ est $C_1$.

13. La composition selon la revendication 10 caractérisée en ce que $R_2$ et $R_3$ sont combinés et représentent un cycle à six atomes de carbone.

14. La composition selon la revendication 9 caractérisée en ce que $R_1$ est $C_1$.

**Patentansprüche**

1. Zusammensetzung von alkylsubstituiertem Toluoldiisocyanat, in der der Alkylsubstituent ein tertiäres Kohlenstoffatom aufweist und die Zusammensetzung den folgenden Formeln entspricht:

Formel 1

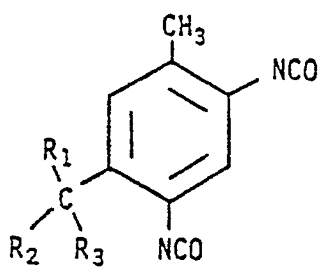

Formel 2

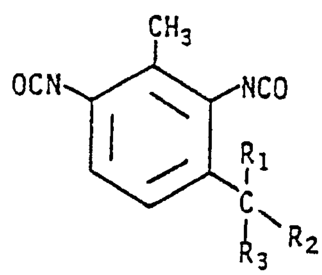

in der $R_1$, $R_2$ und $R_3$ $C_{1-3}$-Alkylgruppen bedeuten oder $R_2$ und $R_3$ zur Bildung eines $C_{5-6}$-gliedrigen Rings kombiniert sind.

2. Zusammensetzung nach Anspruch 1, in der $R_1$, $R_2$ und $R_3$ $C_{1-3}$-Alkylgruppen bedeuten.

3. Zusammensetzung nach Anspruch 2, in der die Zusammensetzung 2,6-Diisocyanato-3-tert-butyltoluol oder 2,4-Diisocyanato-5-tert-butyltoluol ist.

4. Zusammensetzung nach Anspruch 2 enthaltend etwa 65 bis 80 Gew.-% 2,4-Diisocyanato-5-tert-butyltoluol und 20 bis 35 Gew.-% 2,6-Diisocyanato-3-tert-butyltoluol.

5. Zusammensetzung nach Anspruch 4, in der die Zusammensetzung etwa 80 Gew.-% 2,4-Diisocyanato-5-tert-butyltoluol und 20 Gew.-% 2,6-Diisocyanato-3-tert-butyltoluol enthält.

6. Zusammensetzung nach Anspruch 2, in der $R_1$ Ethyl ist und $R_2$ Methyl oder Ethyl ist und $R_3$ eine Methylgruppe ist.

7. Zusammensetzung nach Anspruch 6, in der $R_1$ Ethyl, $R_2$ Ethyl und $R_3$ Methyl ist.

8. Zusammensetzung nach Anspruch 2, in der $R_1$ Methyl ist und $R_2$ und $R_3$ Methyl- oder Ethylgruppen sind.

9. Zusammensetzung nach Anspruch 2, in der $R_1$ Ethyl, $R_2$ Methyl oder Ethyl und $R_3$ Methyl ist.

10. Zusammensetzung nach Anspruch 1, in der $R_1$ eine $C_{1-3}$-Alkylgruppe ist und $R_2$ und $R_3$ zu einem Ring kombiniert sind, der 5 bis 6 Kohlenstoffatome enthält und die Zusammensetzung etwa 65 bis 80 Gew.-% der Formel 1 und etwa 20 bis 35 Gew.-% der Formel 2 enthält.

11. Zusammensetzung nach Anspruch 10, in der $R_2$ und $R_3$ zur Bildung eines 5 Kohlenstoffatome enthaltenden Rings kombiniert sind.

12. Zusammensetzung nach Anspruch 11, in der $R_1$ $C_1$ ist.

13. Zusammensetzung nach Anspruch 10, in der $R_2$ und $R_3$ kombiniert sind und einen 6 Kohlenstoffatome-haltigen Ring bedeuten.

14. Zusammensetzung nach Anspruch 9, in der $R_1$ $C_1$ ist.